# FASCICULE DE BREVET EUROPEEN

(11) **EP 4 419 014 B1**
(45) Date de publication et mention de la délivrance du brevet: **03.12.2025**
(21) Numéro de dépôt: 22803240.5
(22) Date de dépôt: 18.10.2022
(51) Int. Cl.: A61B 17/00, G05G 1/40

(54) **SUPPORT DE POSITIONNEMENT DE PÉDALES DE COMMANDES D'OUTILS D'INTERVENTION MEDICO-TECHNIQUE OU CHIRURGICALE**
HALTERUNG ZUR POSITIONIERUNG VON STEUERPEDALEN VON MEDIZINTECHNISCHEN ODER CHIRURGISCHEN EINGRIFFSWERKZEUGEN
SUPPORT FOR POSITIONING CONTROL PEDALS OF MEDICAL-TECHNICAL OR SURGICAL INTERVENTION TOOLS

(30) Priorité: 20.10.2021 FR 2111134
(43) Date de publication de la demande: 28.08.2024
(73) Titulaire: Hospices Civils de Lyon, 69229 Lyon Cedex 02 (FR); Université Claude Bernard Lyon 1, 69100 Villeurbanne (FR)
(72) Inventeur: PIOCHE, Mathieu, 69380 Belmont d'Azergues (FR); RIVORY, Jérôme, 69390 Vourles (FR); MOCHET, Mikael, 69500 Bron (FR); VIRELY, Mélia, 21320 Sainte-Sabine (FR)
(74) Mandataire: Opilex
(86) Numéro de dépôt international: PCT/EP2022/078901
(87) Numéro de publication internationale: WO 2023/066894

(56) Documents cités:
- EP-A1- 3 506 044
- WO-A1-2014/175846
- JAY: "Stabiler 4 Monitor halter der mehr kann als drauf steht.", 26 August 2018 (2018-08-26), pages 1 - 3, XP055929322, Retrieved from the Internet <URL:https://www.amazon.de/gp/customer-reviews/R29BB9R95VZGZ9/ref=cm_cr_dp_d_rvw_ttl?ie=UTF8&ASIN=B01BGZRWHG> [retrieved on 20220609]
- SISU1AUS PAUL: "Rudder Pedals are pretty easy to make... - Condor", 15 November 2013 (2013-11-15), pages 1 - 3, XP055929251, Retrieved from the Internet <URL:https://www.condorsoaring.com/forums/viewtopic.php?t=15497> [retrieved on 20220609]

## Description

L'invention concerne l'appareillage d'intervention médico-technique ou chirurgicale, et en particulier les équipements commandés par des pédales destinées à être sollicitées par le pied d'un praticien.

Dans le cadre d'une intervention chirurgicale ou médico-technique, un certain nombre d'équipements peuvent être commandés par des pédales, afin que le praticien puisse disposer de ses mains pour d'autres manipulations. A cet effet, des pédales de commande pneumatiques ou électriques sont positionnées au niveau des pieds du praticien, afin de pouvoir commander des équipements respectifs.

Par exemple, en gastroentérologie, le praticien peut être amené à disposer d'une pédale commandant un outil de coupe, d'une pédale commandant un outil de coagulation, d'une pédale commandant un outil d'injection de liquide conducteur, d'une pédale de prise de vue, d'une pédale de changement de fonction et d'une pédale commandant un jet d'eau au niveau de l'endoscope. Ces pédales provenant d'outils très hétérogènes, leur poids est très variable. Les pédales les plus légères peuvent alors être déplacées malencontreusement lors de leur manipulation. Ainsi, lorsqu'un praticien a les yeux focalisés sur le champ opératoire, il est préférable qu'il n'ait pas à quitter ce champ opératoire des yeux afin de voir où les pédales sont positionnées. Par conséquent, le praticien mémorise la position des pédales pour les actionner.

Si le praticien est amené à déplacer malencontreusement certaines pédales, en particulier les plus légères, il doit alors nécessairement abaisser le regard vers ses pieds pour déterminer où se trouve une pédale qui aurait pu être déplacée. Par ailleurs, le positionnement relatif entre les pédales est relativement mal défini. Ainsi, des praticiens débutants sont davantage amenés à quitter des yeux le champ opératoire pour identifier la position des pédales et éviter une erreur, ce qui peut rallonger sensiblement le temps d'intervention, et faire perdre la position précise de l'acte thérapeutique.

L'invention vise à résoudre un ou plusieurs de ces inconvénients. L'invention porte ainsi sur un support de positionnement de pédales de commande d'outils d'intervention médico-technique ou chirurgicale tel que défini dans la revendication 1 annexée.

L'invention porte également sur les variantes des revendications dépendantes. L'homme du métier comprendra que chacune des caractéristiques des variantes suivantes peut être combinée indépendamment aux caractéristiques ci-dessus, sans pour autant constituer une généralisation intermédiaire.

D'autres caractéristiques et avantages de l'invention ressortiront clairement de la description qui en est faite ci-après, à titre indicatif et nullement limitatif, en référence aux dessins annexés, dans lesquels :
[Fig.1] est une vue en perspective d'un support de positionnement de pédales de commande selon un exemple de mode de réalisation de l'invention ;
[Fig.2] est une vue de dessus d'un tronçon d'un lien allongé du support de la figure 1 ;
[Fig.3] est une vue en perspective d'un tronçon d'un lien allongé du système de la figure 1 ;
[Fig.4] est une vue en perspective d'un premier exemple de dispositif de fixation d'une pédale du support de la figure 1 ;
[Fig.5] est une vue de dessus du dispositif de la figure 4 ;
[Fig.6] est une vue de dessous du dispositif de la figure 4 ;
[Fig.7] est une vue en perspective d'un deuxième exemple de dispositif de fixation d'une pédale du support de la figure 1 ;
[Fig.8] est une vue de dessus du dispositif de la figure 7 ;
[Fig.9] est une vue de dessous du dispositif de la figure 7 ;
[Fig.10] est une vue de dessus d'un logement de réception du support de la figure 1 ;
[Fig.11] est une vue en perspective du logement de réception de la figure 10 ;
[Fig.12] est une vue en perspective d'une cale d'orientation d'un tronçon du support de la figure 1 ;
[Fig.13] est une vue de dessus de cales d'orientation de la figure 12 ;
[Fig.14] est une vue de dessus d'un raccord de fixation au sol du support de la figure 1 ;
[Fig.15] est une vue en perspective du raccord de la figure 14 ;
[Fig.16] est une vue en perspective du support de positionnement de la figure 1 dans une autre configuration ;
[Fig. 17] est une vue en perspective d'un support de positionnement de pédales de commande selon un deuxième exemple de mode de réalisation de l'invention ;
[Fig. 18] est une vue en perspective d'un deuxième logement de réception du support de la figure 17 ;
[Fig. 19] est une vue en perspective de deux tronçons intermédiaires directs du lien allongé du système de la figure 17, accouplés au deuxième logement de réception selon la figure 18 ;
[Fig. 20] est une vue en perspective d'une partie d'un support de positionnement de pédales de commande selon un troisième exemple de mode de réalisation de l'invention ;
[Fig. 21] est une vue en perspective d'un tronçon intermédiaire du lien allongé du système de la figure 20 ;
[Fig. 22] est une vue en perspective d'une première variante de réalisation du raccord de la figure 14 équipé de passe-câbles ;
[Fig. 23] est une vue en perspective d'une deuxième variante de réalisation du raccord de la figure 14 équipé de passe-câbles.

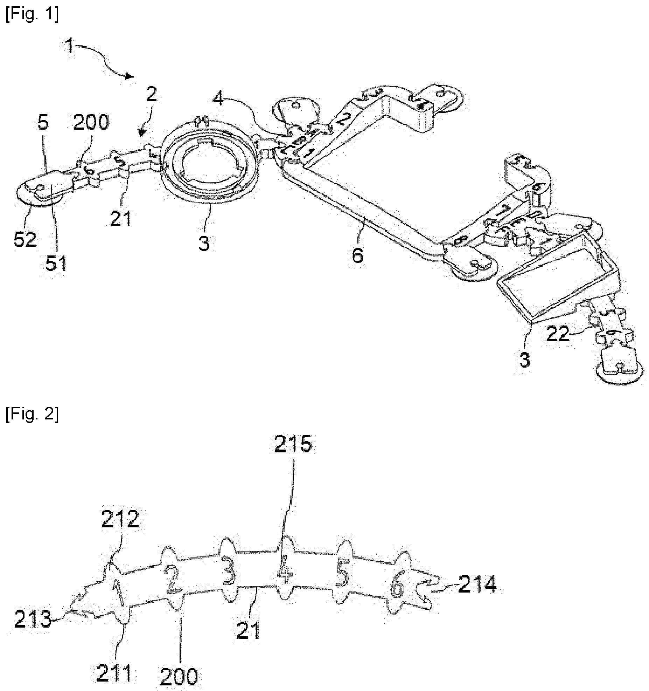

La figure 1 est une vue en perspective d'un support de positionnement 1 de pédales de commande (non illustrées) selon un exemple de mode de réalisation de l'invention. Une pédale de commande d'outil d'intervention médico-technique ou chirurgicale comporte typiquement un actionneur mécanique, dont la sollicitation actionne un contacteur renvoyant un signal de commande par l'intermédiaire d'un flexible pneumatique, hydraulique ou électrique. L'invention vise à la fois à maintenir en position plusieurs pédales pouvant être associés à des outils hétérogènes, et à permettre de modifier les positions relatives des différentes pédales pour s'adapter aux besoins du praticien et individualiser ces positions.

En particulier, une étude prospective randomisée pour évaluer les avantages du support de positionnement 1 selon l'invention a été réalisée dans cinq centres français d'expertise en dissection sous-muqueuse endoscopique, à savoir quatre hôpitaux universitaires et un hôpital privé. Huit médecins ont été impliqués.

Au cours des trois mois d'évaluation, les procédures de dissection sous-muqueuse endoscopique ont été randomisées pour répartir la procédure dans les deux groupes d'étude suivant :
- Un groupe de référence réalisant une procédure standard avec quatre pédales agencées libres les unes par rapport aux autres, dont un bloc d'unité électrochirurgicale de deux pédales, une pour la coupe et une pour la coagulation, une pédale de pompe de rinçage, et une pédale de pompe d'injection pour le couteau ;
- Un groupe test réalisant une procédure avec les quatre mêmes pédales que le groupe de référence à la différence que ces pédales sont agencées sur le support de positionnement 1 selon l'invention, le réglage en position des pédales sur ledit support étant laissée à la préférence du médecin réalisant ladite procédure.

Des procédures de dissection sous-muqueuse endoscopique digestives conventionnelles consécutives ont été réalisées, à l'aide d'un ou de plusieurs gastroscopes conventionnels, d'un capuchon à l'extrémité distale, d'un couteau de dissection sous-muqueuse endoscopique (laissé au choix du médecin) et des pédales. L'utilisation d'une stratégie de contre-traction a été laissée à la discrétion de l'opérateur.

L'étude avait pour objectif l'évaluation du nombre d'erreurs de pied au cours de la procédure, en particulier l'appui du pied sur une mauvaise pédale ou l'appui du pied à côté de la pédale. Des critères d'évaluation secondaires ont également été étudiés, en particulier :
- Le nombre de regards vers le bas pour contrôler la position des pédales pendant la procédure ;
- Le nombre de replacements des pédales par le médecin ou son assistant pendant la procédure ;
- L'évaluation subjective du confort pendant la procédure en utilisant une échelle numérique de 0, signifiant inconfortable, à 10, signifiant confort maximal.

Les données ont été présentées sous forme de fréquences et de pourcentages pour les variables catégorielles. Les données normalement distribuées ont été exprimées en moyennes (écarts-types) et les données non normalement distribuées exprimées en médianes (intervalles interquartiles, IQR). Les échantillons associés ont été comparés à l'aide du test non paramétrique de Wilcoxon, une distribution normale standardisée ne pouvant être considérée en raison de la petite taille de l'échantillon. Les tests de Chi Square et de Fisher ont été utilisés pour analyser les données qualitatives. Une valeur statistique p a été calculé et a été considéré comme indiquant une signification statistique lorsque p < 0,05.

Au total, au cours de la période d'étude, 107 procédures ont été effectuées par les huit médecins dont 54 procédures effectuées par le groupe de référence et 53 effectuées par le groupe test. Parmi ces procédures, un médecin en a effectué 20, deux médecins en ont effectué 19, quatre médecins en ont effectué 10 et un médecin en a effectué 9.

La majorité, soit 91,6 %, des procédures ont été réalisées pour des lésions colorectales. Pour chaque procédure, la taille de la lésion est de 50 mm (IQR 40-65) et la durée de la procédure est de 40 minutes (IQR 26-71). Ils n'existaient pas de différence statistique significative entre les deux groupes d'un point de vue de la localisation de la lésion et de la vitesse de dissection sous-muqueuse endoscopique. La durée des procédures a néanmoins été significativement plus courte pour le groupe test, avec une durée moyenne de 35 minutes, que pour le groupe de référence avec une durée moyenne de 50 min (p = 0. 039). Le nombre médian d'erreurs par heure de procédure était de 0,0/h dans le groupe test et de 1,9/h dans le groupe de référence (p<0,001). Le nombre de regards vers le bas pour contrôler la position des pédales était de 2,2/h pour le groupe test et de 7,7/h pour le groupe de référence (p<0,001). Le nombre médian de replacements des pédales par le médecin ou l'infirmière était de 0,0/h dans le groupe test et 1,7/h dans le groupe de référence (p<0,001). L'évaluation subjective du confort de la commande des pédales pendant la procédure était de 9/10 pour le groupe test contre 7/10 pour le groupe de référence (p = 0,015).

L'étude démontre ainsi l'efficacité du support de positionnement 1 selon l'invention dans la réduction des erreurs opérationnelles et dans la diminution des temps de procédure.

Les pédales de commande peuvent par exemple correspondre à une pédale commandant un outil de coupe, une pédale commandant un outil de coagulation, une pédale commandant un outil d'injection de liquide conducteur, une pédale de prise de vue, une pédale de changement de fonction et une pédale commandant un jet d'eau au niveau de l'endoscope. Ces pédales peuvent être associées à des outils très hétérogènes et présenter des facteurs de forme très hétérogènes. Leur poids peut notamment être très variable.

A cet effet, un support de positionnement 1 selon l'invention comporte un lien allongé 2. Le lien allongé 2 s'étend selon une direction par exemple définie par une droite passant par ses deux extrémités, cette direction définissant le sens de la longueur du lien 2. En effet, le lien allongé 2 peut être rectiligne ou curviligne. Dans l'exemple de la figure 1, le lien allongé 2 s'étend avantageusement selon un arc de cercle. Le lien allongé 2 est muni de plusieurs emplacements de fixation 200 répartis sur sa longueur.

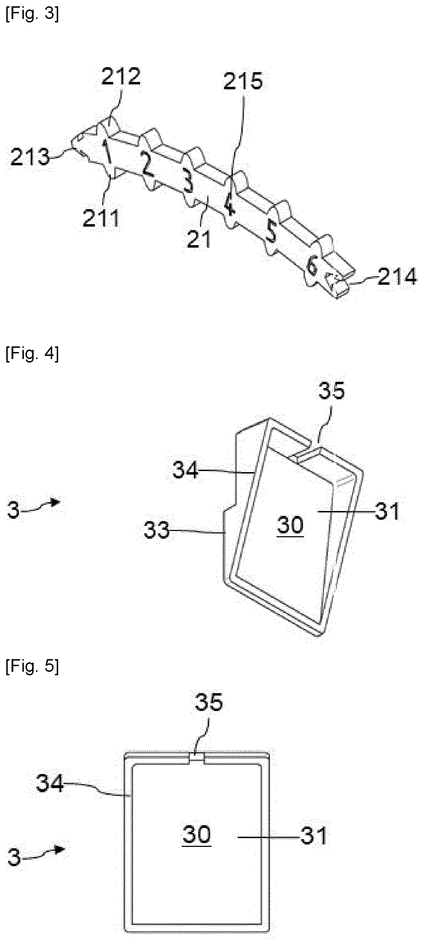

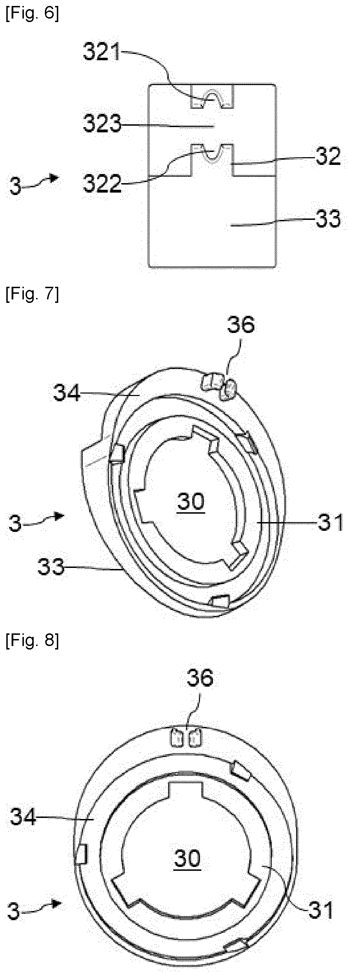

Le support de positionnement 1 comporte plusieurs dispositifs de fixation 3 de pédales de commande. Un premier exemple de dispositif de fixation 3 est illustré aux figures 4 à 6. Un autre exemple de dispositif de fixation 3 est illustré aux figures 7 à 9. Les dispositifs de fixation 3 comprennent chacun une première interface 31 pour l'emboîtement d'une pédale de commande afin de maintenir celle-ci en position. Cette première interface 31 est configurée pour maintenir en position une pédale de commande d'un facteur de forme donné, et ainsi éviter que celle-ci ne puisse être déplacée malencontreusement par un praticien. La présence de plusieurs dispositifs de fixation 3 permet ainsi de s'adapter à différentes configurations de pédales de commande distribuées dans le commerce. Les dispositifs de fixation 3 comprennent chacun une deuxième interface 32 de solidarisation à un emplacement de fixation 200 du lien allongé 2. La deuxième interface de solidarisation 32 est configurée pour pouvoir être sélectivement solidarisée ou désolidarisée d'un emplacement 200 du lien allongé 2. Ainsi, les dispositifs de fixation 3 peuvent être sélectivement positionnés à des positions respectives souhaitées par différents praticiens utilisant successivement le support de positionnement 1. Par ailleurs, comme le lien 2 permet de solidariser plusieurs pédales de commande, chaque pédale de commande ajoutée, par son poids propre, contribue à un maintien stable du support de positionnement 1.

Avantageusement, le lien allongé 2 comporte au moins 8 emplacements 200 répartis sur sa longueur, de préférence au moins 10 emplacements 200, afin de favoriser un positionnement précis des dispositifs de fixation 3 ou de permettre la fixation d'un nombre relativement important de ces dispositifs de fixation 3. Afin de pouvoir aisément positionner des dispositifs de fixation 3 en différents emplacements de façon interchangeable, les emplacements de fixation 200 ont avantageusement une forme identique. Afin de favoriser un espacement entre les pédales et la réception d'un nombre suffisant de pédales, le lien allongé 2 présente avantageusement une longueur d'au moins 400 mm.

Dans l'exemple de la figure 1, le lien allongé 2 comporte avantageusement plusieurs tronçons 21 et 22 séparables, tels qu'illustrés en référence aux figures 2 et 3. Les tronçons 21 et 22 sont munis d'une interface d'accouplement 213 à une extrémité longitudinale. Les tronçons 21 et 22 sont également munis d'une interface d'accouplement 214 à l'autre extrémité longitudinale. L'interface d'accouplement 213 est ici de type mâle et peut s'accoupler soit à une interface d'accouplement 214 complémentaire (ici de type femelle) d'un autre tronçon, soit à une interface d'accouplement complémentaire (ici de type femelle) d'un logement de réception 6 détaillé par la suite.

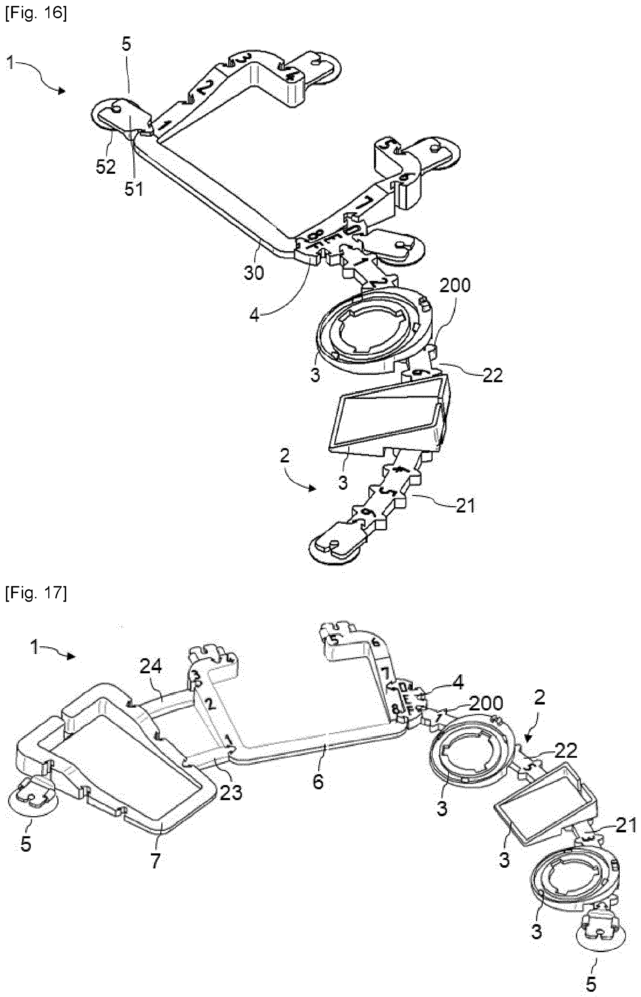

Dans la configuration illustrée à la figure 1, le lien allongé 2 s'étend de part et d'autre d'un logement de réception 6. Les tronçons 21 et 22 sont ici assemblés au logement de réception 6 pour former un lien allongé 2 avec celui-ci. Dans la configuration illustrée à la figure 16, les tronçons 21 et 22 sont assemblés pour former un lien allongé continu 2, ici d'un même côté d'un logement de réception 6.

Avantageusement, le lien 2 et/ou les tronçons 21 et 22 sont non rectilignes et suivent un arc de cercle. Un tel arc de cercle présente avantageusement un rayon de courbure compris entre 0,2 et 0,5 mètres, qui facilite un passage d'une pédale à une autre par un simple pivotement autour du talon du praticien.

Avantageusement, le lien 2 et/ou les tronçons 21 et 22 ont des emplacements de fixation 200 définis par des ergots 211, 212 en saillie transversalement, selon une direction perpendiculaire à la direction d'extension du lien 2. Les ergots 211 et 212 peuvent être formés en saillie par rapport à un corps présentant typiquement une largeur comprise entre 20 et 40 mm. Le corps peut être formé en matériau synthétique.

Avantageusement, les tronçons 21 et 22 présentent un plan de symétrie incluant la direction d'extension du lien. Ainsi, un tronçon 21 ou 22 peut être placé indifféremment de chaque côté d'un logement de réception 6.

Avantageusement, le lien allongé 2 et/ou les tronçons présentent des indexations différentes 215 au niveau de chacun desdits emplacements de fixation 200. Ainsi, un praticien pourra mémoriser la position de différents dispositifs de fixation 3 le long du lien 2, afin de disposer d'une configuration reproductible de la position des pédales de commande.

Les dispositifs de fixation 3 des figures 4 à 6 et des figures 7 à 9 comportent une première surface 33 destinée à entrer en contact avec le sol et une deuxième surface 34 destinée à entrer en contact avec une pédale de commande. La deuxième surface 34 est avantageusement inclinée par rapport à la première surface 33 d'un angle d'au moins 10°, ce qui permet d'incliner une pédale vers un praticien pour une ergonomie améliorée. La surface 34 est ici formée au sommet d'une paroi ceinturant un volume de réception 30 d'une pédale.

Dans l'exemple des figures 4 à 6, le dispositif de fixation 3 comporte une interface de solidarisation 32 présentant des encoches 321 et 322 positionnées en vis-à-vis et dont la forme est complémentaire de celle des ergots 211 et 212 du lien allongé 2. Les encoches 321 et 322 sont séparées par un espace 323 permettant le passage du corps du lien allongé 2.

Dans l'exemple des figures 4 à 6, une fente 35 est avantageusement ménagée dans la paroi pour permettre le passage et l'immobilisation d'un flexible d'une pédale de commande.

Dans l'exemple des figures 7 à 9, un étrier 36 est avantageusement ménagé au sommet de la paroi pour permettre le passage et l'immobilisation d'un flexible d'une pédale de commande.

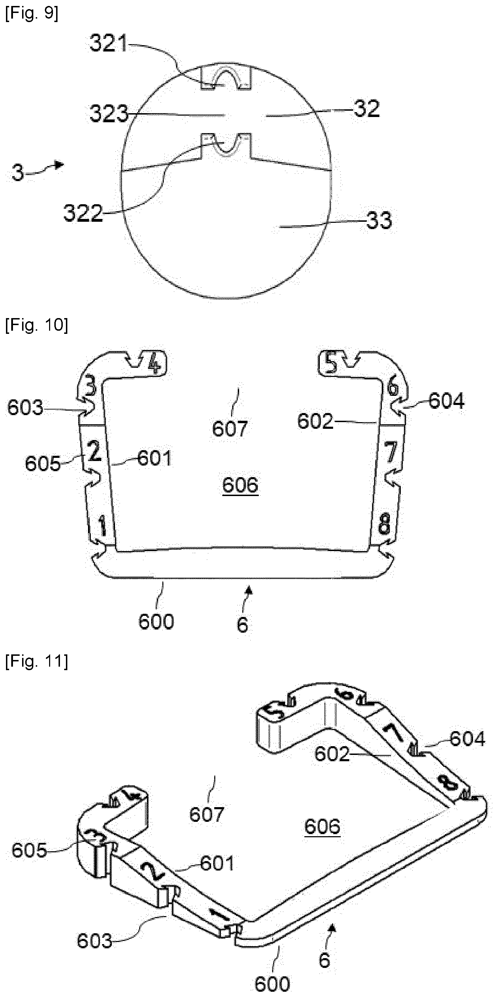

Le support 1 illustré à la figure 1 comporte avantageusement un logement de réception 6 d'une pédale, fixé dans l'alignement du lien allongé 2 et illustré aux figures 10 et 11. Le logement de réception 6 est typiquement destiné à recevoir un bloc de commande comportant plusieurs pédales. Un tel bloc de commande peut permettre de faciliter le maintien en position du support 1, par son poids.

Le logement de réception 6 comporte deux interfaces d'accouplement 603 et 604. Les interfaces d'accouplement 603 et 604 sont complémentaires de l'interface d'accouplement 213 des tronçons 21 et 22. Les interfaces d'accouplement 603 et 604 sont positionnées au niveau d'extrémités opposées 601 et 602 du logement de réception 6. Le logement de réception 6 se présente ici sous la forme d'un étrier 600 en forme de U. L'étrier 600 délimite un espace 606 destiné à recevoir une pédale ou un bloc de commande, et une ouverture 607 permettant le passage de flexibles de la pédale ou bloc de commande.

Avantageusement, comme dans l'exemple illustré aux figures 10 et 11, les extrémités opposées 601 et 602 du logement de réception 6 comportent chacune plusieurs interfaces d'accouplement 603 et 604 respectivement. Ces interfaces d'accouplement 603 et 604 sont espacées selon une direction transversale. Le logement 6 permet ainsi la fonction d'un lien 2 ou de tronçons 21, 22 à différents niveaux transversalement.

En présence de plusieurs interfaces d'accouplement 603 et 604, le logement de réception 6 présente avantageusement des indexations 605 pour chacune d'entre elles. Ainsi, un praticien pourra mémoriser la position du lien 2 ou de tronçons 21 et 22 autour du logement 6.

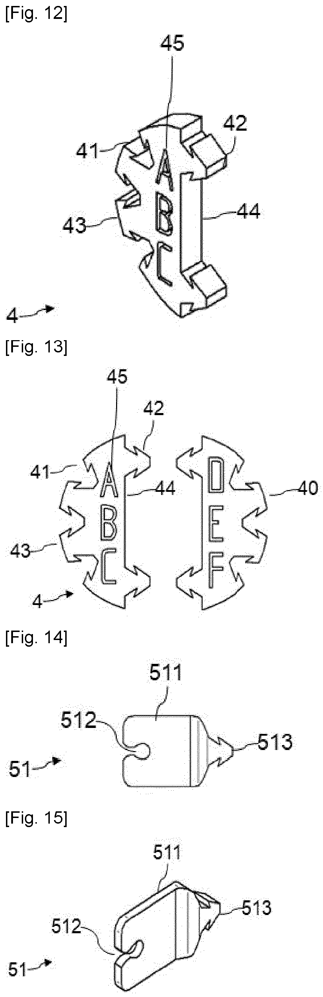

Avantageusement, le support 1 peut être muni d'une ou plusieurs cales amovibles 4, interposées entre le logement 6 et un lien 2 ou tronçon 21, 22. Un exemple de cale amovible 4 est illustré à la figure 12, une paire de cales amovibles 4 et 40 étant illustrée à la figure 13. Une telle cale amovible 4 comporte une ou plusieurs interfaces d'accouplement 41, complémentaires (donc ici de type femelle) de l'interface d'accouplement 213 d'un lien 2 ou d'un tronçon 21. Ces interfaces 41 sont positionnées au niveau d'une extrémité 43 de la cale 4. La cale amovible 4 comporte également une ou plusieurs interfaces d'accouplement 42 complémentaires (donc ici de type mâle) de l'interface d'accouplement 603 ou 604 du logement de réception 6. Ces interfaces 42 sont positionnées au niveau d'une extrémité opposée 44 de la cale 4. Les interfaces d'accouplement 41 et 42 sont configurées de sorte que l'orientation d'un tronçon 21 fixé à une cale 4, elle-même fixée au logement de réception 6 est différente de l'orientation de ce même tronçon 21 fixé directement au logement de réception 6. Ainsi, la cale 4 permet de modifier l'angle selon lequel un tronçon 21 s'étend depuis le logement de réception 6. La cale 4 peut comporter des indexations 45 pour chacune des interfaces d'accouplement 41 ou 42.

Le support de positionnement 1 comporte avantageusement des moyens favorisant sa solidarisation ou son adhérence avec le sol. On peut par exemple prévoir que le lien allongé 2 présente une face présentant un état de surface anti-dérapant. Dans l'exemple illustré à la figure 1, le support 1 est avantageusement muni de plusieurs dispositifs 5 de solidarisation au sol. Un tel dispositif de solidarisation 5 comprend ici des ventouses 52, solidarisables soit au lien allongé 2, soit au logement de réception 6.

Dans l'exemple illustré aux figures 1 et 16, chaque ventouse 52 est accouplée avec un lien respectif 51. Un exemple de lien 51 est illustré en référence aux figures 14 et 15. Le lien 51 comporte avantageusement une interface femelle 512 sous la forme d'un évidement destiné à recevoir un arbre de fixation d'une ventouse 52. Cette interface femelle 512 est fixée à une extrémité d'un corps 511. Le lien 51 comporte avantageusement une interface d'accouplement 513 (ici de type mâle) à une extrémité opposée à l'interface femelle 512. L'interface d'accouplement 513 a une forme complémentaire des interfaces d'accouplement 214, 603, 604 ou 41, afin de pouvoir solidariser le support 1 au sol en différents points.

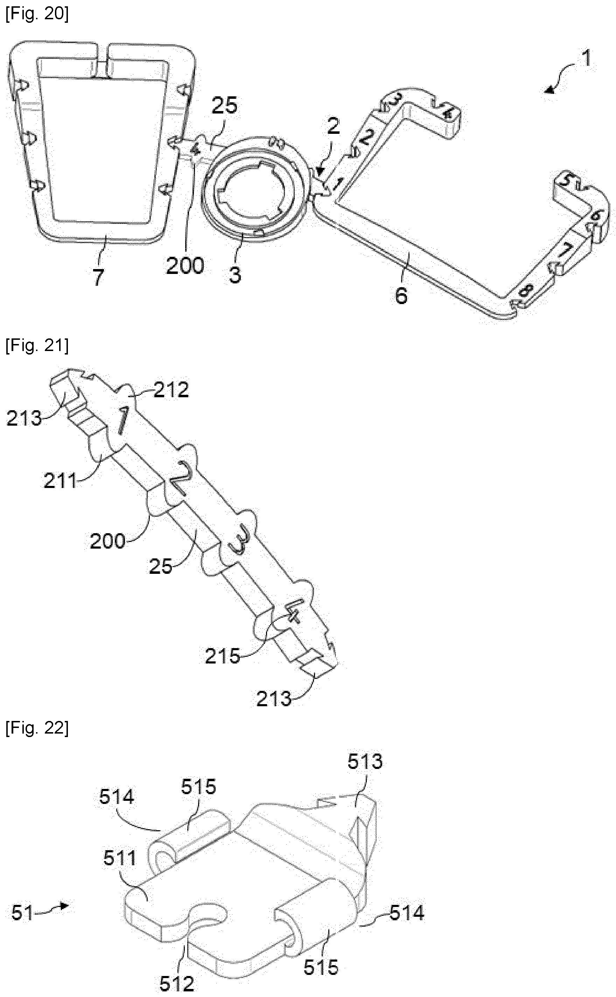

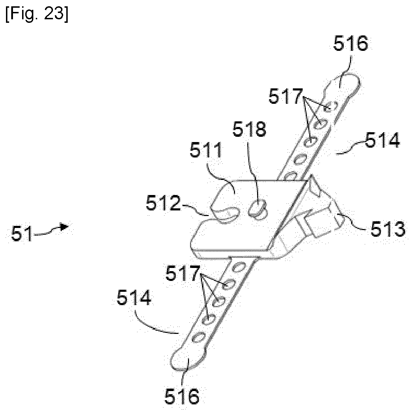

Le dispositif de solidarisation 5 peut également être équipé d'un ou plusieurs passe-câbles 514. Un passe-câble 514 est configuré pour recevoir un ou plusieurs câbles ou flexibles en provenance d'une ou plusieurs pédales de commande. En particulier, et comme représenté sur les figures 22 et 23, le passe-câble 514 peut être agencé sur le lien 51 du dispositif de solidarisation 5.

Dans un premier exemple de réalisation d'un dispositif de solidarisation 5 équipé de passe-câbles représenté sur la figure 22, le passe-câble 514 peut comporter une bande recourbée 515 formant un logement cylindrique. Ce dernier est destiné à recevoir un câble ou flexible en provenance d'une pédale de commande. À ce titre, le logement cylindrique peut avoir un diamètre interne égale, ou supérieures d'au maximum 5 mm, au diamètre du câble ou flexible qu'il est destiné à recevoir. En pratique, le logement cylindrique peut avoir un diamètre de 3 mm à 20 mm. Préférentiellement, le logement cylindrique peut avoir un diamètre de 6,2 mm. La bande recourbée 515 peut avoir une largeur comprise entre 5 mm et 40 mm, une longueur comprise entre 6 mm et 45 mm et une épaisseur entre 0,5 mm et 5 mm. La bande recourbée 515 peut être réalisée en élastomères thermoplastiques (TPE). La bande recourbée 515 est préférentiellement réalisée en polyuréthane thermoplastique (TPU). La bande recourbée 515 peut notamment être réalisée dans un matériau dont le module d'Young est compris entre 46 et 49 MPa. La bande recourbée 515 peut comporter une première extrémité fixée au corps 511 du lien 51. La bande recourbée 515 peut comporter une deuxième extrémité libre écartée du corps 51 de sorte à former un passage. Ce dernier est destiné à permettre l'insertion d'un câble ou flexible dans le logement cylindrique.

Dans un deuxième exemple de réalisation d'un dispositif de solidarisation 5 équipé de passe-câbles représenté sur la figure 23, le passe-câble 514 peut comporter au moins une bande enroulable 516. Cette dernière peut avoir une largeur comprise entre 5 mm et 40 mm, une longueur comprise entre 20 mm et 100 mm et une épaisseur entre 0,5 mm et 5 mm. Dans un mode de réalisation préféré du passe-câble 514, la bande enroulable 516 peut avoir une épaisseur égale à 0,8 mm et une longueur supérieure ou égale à 50 mm. La bande enroulable 516 peut être réalisée en élastomères thermoplastiques (TPE). La bande enroulable 516 est préférentiellement réalisée en polyuréthane thermoplastique (TPU). La bande enroulable 516 peut notamment être réalisée dans un matériau dont le module d'Young est compris entre 46 et 49 MPa. La bande enroulable 516 peut comporter une première extrémité fixée au corps 511 du lien 51. La bande enroulable 516 peut comporter une deuxième extrémité libre. La bande enroulable 516 peut comporter un ou plusieurs orifices 517 agencés le long de ladite bande enroulable. Chaque orifice 517 peut comporter une section de forme circulaire, ovale, carrée, rectangulaire, ou toute autre forme convenant à l'homme du métier. Les orifices 517 peuvent tous avoir la même forme de section, ou au contraire, peuvent avoir des sections de forme différentes. Chaque orifice 517 peut avoir une section dont la plus petite dimension est comprise entre 2 mm et 15 mm. Dans le mode de réalisation préféré du passe-câble 514, chaque orifice 517 peut avoir une section circulaire de diamètre égale à 5 mm.

Le passe-câble 514 peut également comporter une ou plusieurs ancres 518 agencées en saillie à la surface corps 511 du lien 51. L'ancre 518 peut par exemple présenter une partie centrale de forme cylindrique de section de forme circulaire, ovale, carrée, rectangulaire, ou toute autre forme convenant à l'homme du métier. La partie centrale peut avoir une section dont la plus petite dimension est comprise entre 2 mm et 15 mm. Dans le mode de réalisation préféré du passe-câble 514, la partie centrale peut avoir une section circulaire de diamètre égale à 4,8 mm. La partie centrale peut avoir une longueur comprise entre 1 mm et 10 mm. Dans le mode de réalisation préféré du passe-câble 514, la partie centrale peut avoir une longueur de 2,8 mm. La partie centrale peut également présenter une première extrémité fixée sur le corps 511 du lien 51. La partie centrale peut encore présenter une seconde extrémité sur laquelle est aménagé un obstacle. Ce dernier peut par exemple se présenter sous la forme d'un bourrelet, d'un collet, d'un épaulement, ou de toute autre forme d'obstacle convenant à l'homme du métier. Au moins un orifice 517 de la bande enroulable 516 peut alors être configuré pour permettre l'insertion de l'ancre 518 lorsque ladite bande enroulable est repliée sur ladite ancre de sorte à former une boucle à l'intérieur de laquelle peut être agencé au moins un câble ou flexible en provenance d'une ou plusieurs pédales de commande. L'orifice 517 de la bande enroulable 516 peut alors avoir une section de dimensions préférablement supérieures aux dimensions de la section de la partie centrale de l'ancre 518, et inférieures aux dimensions de la section de l'obstacle de ladite ancre. L'orifice 517 de la bande enroulable 516 peut momentanément être déformé pour pouvoir permettre le passage de l'obstacle de l'ancre 518 puis revenir à ses dimensions initiales de sorte à rester en position sur la partie centrale de ladite ancre après franchissement dudit obstacle. Le support 1 peut également comprendre un deuxième logement de réception 7 d'une pédale. Des exemples de réalisation du support 1 comportant un deuxième logement de réception 7 d'une pédale sont notamment représentés aux figures 17 et 20.

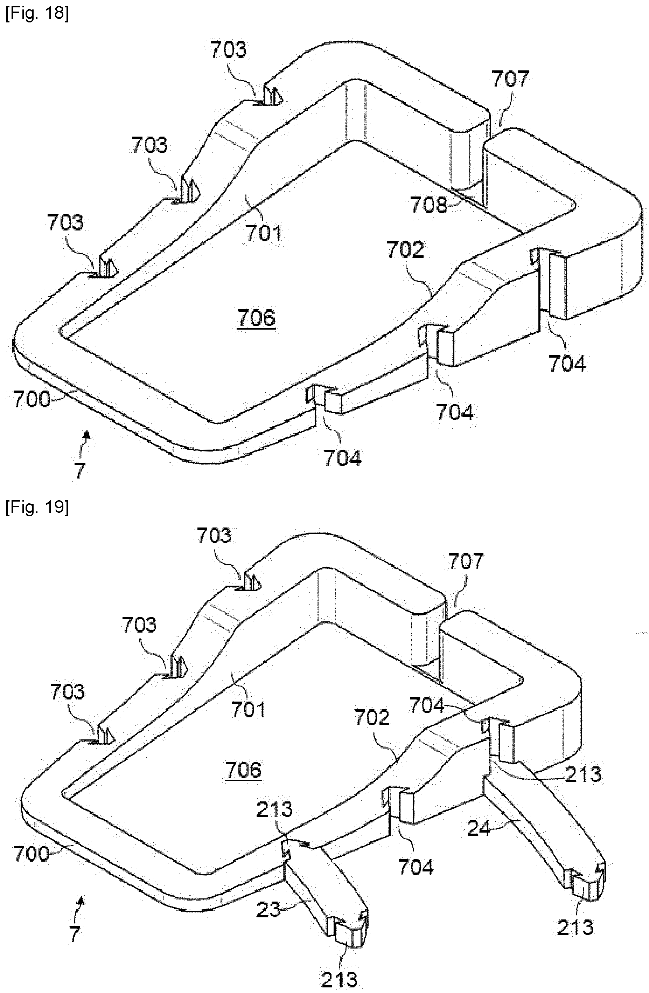

Le deuxième logement de réception 7 peut être relié au premier logement de réception 6, au moyen d'un ou plusieurs tronçons intermédiaires directs 23, 24 du lien allongé 2. Ainsi, le lien allongé 2 peut comporter un tronçon 21 et/ou un tronçon 22 et/ou un ou plusieurs tronçons intermédiaires directs 23, 24. Dans l'exemple de réalisation représenté sur les figures 17 et 19, le lien allongé 2 comporte deux tronçons intermédiaires directs 23, 24 reliant le premier logement de réception 6 au deuxième logement de réception 7 et deux tronçons 21 et 22.

Le tronçon intermédiaire direct 23 ou 24 peut être muni d'une interface d'accouplement 213 à une première extrémité longitudinale. Le tronçon intermédiaire direct 23 ou 24 peut également être muni d'une interface d'accouplement 213 à une deuxième extrémité longitudinale, ladite interface d'accouplement étant identique à l'interface d'accouplement 213 de la première extrémité longitudinale. L'interface d'accouplement 213 peut s'accoupler soit à une interface d'accouplement 214 complémentaire d'un tronçon 21 ou 22, soit à une interface d'accouplement complémentaire 603 ou 604 du premier logement de réception 6 décrit précédemment, soit à une interface d'accouplement complémentaire 703 ou 704 du deuxième logement de réception 7 décrit plus en détail dans la suite de la description. Dans l'exemple de réalisation représenté sur les figures 17 et 19, l'interface d'accouplement 213 du tronçon intermédiaire direct 23 ou 24 est de type mâle et l'interface d'accouplement complémentaire de type femelle.

Avantageusement, et de façon similaire aux tronçons 21 et/ou 22, le tronçon intermédiaire direct 23, ou 24 est non rectiligne et suit un arc de cercle. Un tel arc de cercle présente avantageusement un rayon de courbure compris entre 0,2 m et 0,5 m, qui facilite un passage d'une pédale à une autre par un simple pivotement autour du talon du praticien. Lorsque le lien allongé 2 comporte deux tronçons intermédiaires directs 23, 24 reliant le premier logement de réception 6 au deuxième logement de réception 7, et que lesdits tronçons intermédiaires directs suivent un arc de cercle, lesdits tronçons intermédiaires directs ont avantageusement le même centre de courbure. Le tronçon intermédiaire direct 24 le plus éloigné du centre de courbure a alors une longueur supérieure à la longueur du tronçon intermédiaire direct 23 le plus proche du centre de courbure de sorte que les deux dits tronçons intermédiaires directs s'étendent selon un même secteur angulaire.

Contrairement aux tronçons 21 et/ou 22 précédemment décrits, le tronçon intermédiaire direct 23 ou 24 est dépourvu d'emplacement de fixation et d'indexation.

Le deuxième logement de réception 7 peut également être relié au premier logement de réception 6, par l'intermédiaire d'un tronçon intermédiaire 25 du lien allongé 2. Ainsi, le lien allongé 2 peut comporter un tronçon 21 et/ou un tronçon 22 et/ou un tronçon intermédiaire direct 25. Dans l'exemple de réalisation partiellement représenté sur la figure 20, le lien allongé 2 comporte un tronçon intermédiaire 25 reliant le premier logement de réception 6 au deuxième logement de réception 7 ainsi que deux tronçons 21 et 22 non représentés, mais similaires aux tronçons 21 et 22 représentés sur la figure 16. Un exemple de tronçon intermédiaire 25 est représenté en détail sur la figure 21.

Le tronçon intermédiaire 25 peut être muni d'une interface d'accouplement 213 à une première extrémité longitudinale. Le tronçon intermédiaire 25 peut également être muni d'une interface d'accouplement 213 à une deuxième extrémité longitudinale, ladite interface d'accouplement étant identique à l'interface d'accouplement 213 de la première extrémité longitudinale. L'interface d'accouplement 213 peut s'accoupler soit à une interface d'accouplement 214 complémentaire d'un tronçon 21 ou 22, soit à une interface d'accouplement complémentaire 603 ou 604 du premier logement de réception 6 décrit précédemment, soit à une interface d'accouplement complémentaire 703 ou 704 du deuxième logement de réception 7 décrit plus en détail dans la suite de la description. Dans l'exemple de réalisation représenté sur les figures 20 et 21, l'interface d'accouplement 213 du tronçon intermédiaire 25 est de type mâle et l'interface d'accouplement complémentaire de type femelle.

Avantageusement, et de façon similaire aux tronçons 21 et/ou 22, le tronçon intermédiaire 25 est non rectiligne et suit un arc de cercle. Un tel arc de cercle présente avantageusement un rayon de courbure compris entre 0,2 m et 0,5 m, qui facilite un passage d'une pédale à une autre par un simple pivotement autour du talon du praticien.

Avantageusement, le tronçon intermédiaire 25 a des emplacements de fixation 200 définis par des ergots 211, 212 en saillie transversalement, selon une direction perpendiculaire à la direction d'extension du lien 2. Les ergots 211 et 212 peuvent être formés en saillie par rapport à un corps présentant typiquement une largeur comprise entre 20 et 40 mm. Le corps peut être formé en matériau synthétique.

Avantageusement, le tronçon intermédiaire 25 présente des indexations différentes 215 au niveau de chacun desdits emplacements de fixation 200. Ainsi, un praticien pourra mémoriser la position de différents dispositifs de fixation 3 le long du lien 2, afin de disposer d'une configuration reproductible de la position des pédales de commande.

Le deuxième logement de réception 7 est typiquement destiné à recevoir un bloc de commande comportant une pédale. Un tel bloc de commande peut permettre de faciliter le maintien en position du support 1, par son poids. Dans les exemples de réalisation représentés sur les figures 17 à 20, le deuxième logement de réception 7 peut par exemple être particulièrement adapté à recevoir une pédale de type ERBE monopédale.

Le deuxième logement de réception 7 est du même type que le premier logement de réception 6. Ainsi, et comme représenté sur la figure 18, le deuxième logement de réception 7 peut comporter au moins une interface d'accouplement 704. L'interface d'accouplement 704 est complémentaire soit de l'interface d'accouplement 213 du tronçon intermédiaire direct 23 ou 24, soit de l'interface d'accouplement 213 du tronçon intermédiaire 25 selon le mode de réalisation retenu. L'interface d'accouplement 704 est positionnée au niveau d'une première extrémité 702 du deuxième logement de réception 7. Une deuxième interface d'accouplement 703 peut également être positionnée au niveau d'une deuxième extrémité 701 du deuxième logement de réception 7. La deuxième interface d'accouplement 703 peut être complémentaire à l'interface d'accouplement 213 du tronçon 21 ou 22. Ainsi, il est possible d'accoupler un tronçon 21 ou 22 sur la deuxième extrémité 701 du deuxième logement de réception 7 de façon identique à l'accouplement au premier logement de réception 6 dans le mode de réalisation représenté sur la figure 1. De façon similaire à ce qui a été précédemment décrit pour le premier logement de réception 6, une cale amovible 4 peut être intercalée entre la deuxième interface d'accouplement 703 et le tronçon 21 ou 22. Le deuxième logement de réception 7 peut se présenter sous la forme d'un étrier 700 en forme de U. L'étrier 700 délimite un espace 706 destiné à recevoir une pédale ou un bloc de commande, et une ouverture 707 permettant le passage de flexibles de la pédale ou bloc de commande. L'ouverture 707 peut être aménagée sur toute la hauteur de l'étrier 700. Dans une variante de réalisation représentée sur la figure 18, l'ouverture 707 peut être aménagée sur une partie de la hauteur de l'étrier, une bande de matière 708 reliant les première et deuxième extrémités 701, 702.

Avantageusement, comme représentées sur les figures 17 à 20, les extrémités opposées 701 et 702 du deuxième logement de réception 7 peuvent chacune comporter plusieurs interfaces d'accouplement 703 et 704 respectivement. Ces interfaces d'accouplement 703 et 704 peuvent être espacées selon une direction transversale. Le deuxième logement de réception 7 permet ainsi la fonction d'un lien 2 ou de tronçons 21, 22 ou de tronçons intermédiaires directs 23, 24 ou d'un tronçon intermédiaire 25 à différents niveaux transversalement.

En présence de plusieurs interfaces d'accouplement 703 et 704, le deuxième logement de réception 7 peut avantageusement présenter des indexations, non représentées, pour chacune d'entre elles. Ainsi, un praticien pourra mémoriser la position du lien 2 ou de tronçons 21 et 22 autour du deuxième logement de réception 7.

Les différents composants du support 1 pourront par exemple être réalisés en matériaux synthétiques, afin de permettre leur lavage/étuvage entre plusieurs utilisations.

## Revendications

1. Support de positionnement (1) de pédales de commande d'outils d'intervention médico-technique ou chirurgicale, **caractérisé en ce qu'**il comprend :
- un lien allongé (2) muni de plusieurs emplacements de fixation (200) répartis sur sa longueur, le lien allongé (2) comportant plusieurs tronçons (21, 22) séparables et munis d'une interface d'accouplement (213) à une extrémité ;
- un logement de réception (6) d'une pédale fixé dans l'alignement du lien allongé (2), le logement de réception (6) comportant deux interfaces d'accouplement (603, 604) complémentaires de l'interface d'accouplement (213) desdits tronçons, lesdites interfaces d'accouplement (603, 604) du logement de réception (6) étant positionnées au niveau d'extrémités opposées (601, 602) dudit logement de réception (6) ;
- plusieurs dispositifs de fixation (3) de pédales de commande, comprenant chacun une première interface (31) pour l'emboîtement d'une pédale de commande, et une deuxième interface (32) de solidarisation à un emplacement de fixation (200) du lien allongé (2), la deuxième interface de solidarisation (32) étant configurée pour pouvoir être sélectivement solidarisée ou désolidarisée d'un emplacement du lien allongé (2) ; chaque dispositif (3) de fixation de pédale de commande comportant une première surface (33) destinée à entrer en contact avec le sol lorsque ce dispositif de fixation est solidarisé au lien allongé (2), et une deuxième surface (34) destinée à entrer en contact avec une pédale de commande.

2. Support de positionnement (1) selon la revendication 1, dans lequel lesdites extrémités opposées (601, 602) dudit logement de réception (6) comportent chacune plusieurs desdites interfaces d'accouplement (603) espacées.

3. Support de positionnement (1) selon la revendication 1 ou 2, comprenant en outre une cale amovible (4) présentant une première interface d'accouplement complémentaire (41) de l'interface d'accouplement d'un tronçon séparable (213), et présentant une deuxième interface d'accouplement (42) complémentaire de l'interface d'accouplement (603) du logement de réception (6), les première et deuxième interfaces d'accouplement (41, 42) étant positionnées au niveau d'extrémités opposées (43, 44) de la cale (4), de sorte que l'orientation d'un tronçon (21) fixé à une cale (4) fixée au logement de réception (6) est différente de l'orientation d'un tronçon (21) fixé directement au logement de réception (6).

4. Support de positionnement (1) selon l'une quelconque des revendications 1 à 3, dans lequel les tronçons (21 ,22) présentent un plan de symétrie incluant une direction d'extension du lien (2).

5. Support de positionnement (1) selon l'une quelconque des précédentes, dans lequel le lien (2) présente un rayon de courbure compris entre 0,2 et 0,5 mètres (pour permettre de les atteindre avec talon posé et simple pivotement).

6. Support de positionnement (1) selon l'une quelconque des revendications précédentes, dans lequel le lien allongé (2) présente des indexations différentes (215) au niveau de chacun desdits emplacements de fixation (200).

7. Support de positionnement (1) selon l'une quelconque des revendications précédentes, dans lequel lesdits emplacements de fixation (200) comportent des ergots (211, 212) en saillie selon une direction perpendiculaire à la direction d'extension du lien.

8. Support de positionnement (1) selon l'une quelconque des revendications précédentes, dans lequel lesdits emplacements de fixation (200) ont une forme identique.

9. Support de positionnement (1) selon l'une quelconque des revendications précédentes, dans lequel ledit lien (2) présente une face présentant un état de surface anti-dérapant.

10. Support de positionnement (1) selon l'une quelconque des revendications précédentes, dans lequel la deuxième surface est inclinée par rapport à la première surface d'un angle d'au moins 10°.

11. Support de positionnement (1) selon l'une quelconque des revendications précédentes, dans lequel ledit lien allongé (2) présente une longueur d'au moins 400 mm.

12. Système, incluant :
- un support de positionnement (1) selon l'une quelconque des revendications précédentes ;
- une pédale de commande emboîtée dans une première interface (31) d'un desdits dispositifs de fixation,

## Patentansprüche

1. Positionierungshalterung (1) von Steuerpedalen von medizintechnischen oder chirurgischen Eingriffswerkzeugen, **dadurch gekennzeichnet, dass** sie Folgendes umfasst:
- eine längliche Verbindung (2), die mit mehreren Befestigungsstellen (200) versehen ist, die auf ihre Länge verteilt sind, wobei die längliche Verbindung (2) mehrere Abschnitte (21, 22) umfasst, die trennbar sind und mit einer Kopplungsschnittfläche (213) an einem Ende versehen sind;
- einen Aufnahmeraum (6) eines Pedals, das in Ausrichtung mit der länglichen Verbindung (2) befestigt ist, wobei der Aufnahmeraum (6) zwei Kopplungsschnittflächen (603, 604) umfasst, die zu der Kopplungsschnittfläche (213) der Abschnitte komplementär sind, wobei die Kopplungsschnittflächen (603, 604) des Aufnahmeraums (6) an einander entgegengesetzter Enden (601, 602) des Aufnahmeraums (6) positioniert sind;
- mehrere Befestigungsvorrichtungen (3) von Steuerpedalen, die jeweils eine erste Schnittfläche (31) zum Einpassen eines Steuerpedals umfassen, und eine zweite Schnittfläche (32) zur festen Verbindung an einer Befestigungsstelle (200) der länglichen Verbindung (2), wobei die zweite Befestigungsschnittfläche (32) dazu konfiguriert ist, selektiv fest mit einer Stelle der länglichen Verbindung (2) verbunden oder davon getrennt zu werden; wobei jede Befestigungsvorrichtung (3) eines Steuerpedals eine erste Oberfläche (33) umfasst, die dazu bestimmt ist, mit dem Boden in Berührung zu kommen, wenn diese Befestigungsvorrichtung fest mit der länglichen Verbindung (2) verbunden wird, und eine zweite Oberfläche (34), die dazu bestimmt ist, mit einem Steuerpedal in Berührung zu kommen.

2. Positionierungshalterung (1) nach Anspruch 1, wobei die einander entgegengesetzten Enden (601, 602) des Aufnahmeraums (6) jeweils mehrere der voneinander beabstandeten Kopplungsschnittflächen (603) umfassen.

3. Positionierungshalterung (1) nach Anspruch 1 oder 2, die außerdem einen abnehmbaren Keil (4) umfasst, der eine erste komplementäre Kopplungsschnittfläche (41) der Kopplungsschnittfläche eines trennenbaren Abschnitts (213) aufweist und eine zweite komplementäre Kopplungsschnittfläche (42) der Kopplungsschnittfläche (603) des Aufnahmeraums (6) aufweist, wobei die erste und die zweite Kopplungsschnittfläche (41, 42) an einander entgegengesetzter Enden (43, 44) des Keils (4) derart positioniert sind, dass sich die Ausrichtung eines Abschnitts (21), der an einem Keil (4) befestigt ist, der an dem Aufnahmeraum (6) befestigt ist, von der Ausrichtung eines Abschnitts (21), der direkt an dem Aufnahmeraum (6) befestigt ist, unterscheidet.

4. Positionierungshalterung (1) nach einem der Ansprüche 1 bis 3, wobei die Abschnitte (21, 22) eine Symmetrieebene aufweisen, die eine Erstreckungsrichtung der Verbindung (2) beinhaltet.

5. Positionierungshalterung (1) nach einem der vorstehenden Ansprüche, wobei die Verbindung (2) einen Krümmungsradius zwischen 0,2 und 0,5 Meter aufweist (um zu erlauben, sie bei aufgelegter Ferse und einfachem Schwenken zu erreichen).

6. Positionierungshalterung (1) nach einem der vorstehenden Ansprüche, wobei die längliche Verbindung (2) unterschiedliche Positionierungen (215) auf der Ebene jeder der Befestigungsstellen (200) aufweist.

7. Positionierungshalterung (1) nach einem der vorstehenden Ansprüche, wobei die Befestigungsstellen (200) Dorne (211, 212) umfassen, die gemäß einer Richtung senkrecht zu der Erstreckungsrichtung der Verbindung vorragen.

8. Positionierungshalterung (1) nach einem der vorstehenden Ansprüche, wobei die Befestigungsstellen (200) eine identische Form aufweisen.

9. Positionierungshalterung (1) nach einem der vorstehenden Ansprüche, wobei die Verbindung (2) eine Fläche aufweist, die einen rutschfesten Oberflächenzustand aufweist.

10. Positionierungshalterung (1) nach einem der vorstehenden Ansprüche, wobei die zweite Oberfläche in Bezug auf die erste Oberfläche mit einem Winkel von mindestens 10° geneigt ist.

11. Positionierungshalterung (1) nach einem der vorstehenden Ansprüche, wobei die längliche Verbindung (2) eine Länge von mindestens 400 mm aufweist.

12. System, das Folgendes beinhaltet:
- eine Positionierungshalterung (1) nach einem der vorstehenden Ansprüche;
- ein Steuerpedal, das in einer ersten Schnittfläche (31) einer der Befestigngsvorrichtungen eingepasst ist.

## Claims

1. A positioning support (1) for control pedals for medical-technical or surgical intervention tools, **characterised in that** same comprises:
- an elongated link (2) fitted with a plurality of fastening locations (200) distributed throughout the length thereof, the elongated link (2) having a plurality of sections (21, 22) which are separable and fitted with a coupling interface (213) at one end;
- one receiving housing (6) for a pedal fastened in line with the elongated link (2), the receiving housing (6) having two coupling interfaces (603, 604) matching the coupling interface (213) of said sections, said coupling interfaces (603, 604) of the receiving housing (6) being positioned at opposite ends (601, 602) of said receiving housing (6);
- a plurality of fastening devices (3) for control pedals, each one comprising a first interface (31) for the interlocking of a control pedal, and a second interface (32) for the attachment at a fastening location (200) of the elongated link (2), the second interface for attachment (32) being configured to be selectively attached to or detached from a location of the elongated link (2); each device (3) for fastening a control pedal having a first surface (33) intended to come into contact with the ground when the fastening device is attached to the extended link (2), and a second surface (34) intended to come into contact with a control pedal.

2. The positioning support (1) according to claim 1,
wherein said opposite ends (601, 602) of said receiving housing (6)
each have a plurality of said spaced apart, coupling interfaces (603).

3. The positioning support (1) according to claim 1 or 2, further comprising a removable shim (4) having a first coupling interface (41) matching the coupling interface of a separable section (213), and having a second coupling interface (42) matching the coupling interface (603) of the receiving housing (6), the first and second coupling interfaces (41, 42) being positioned at opposite ends (43, 44) of the shim (4), so that the orientation of a section (21) fastened to a shim (4) fastened to the receiving housing (6) is different from the orientation of a section (21) which is directly fastened to the receiving housing (6).

4. The positioning support (1) according to any of claims 1 to 3, wherein sections (21, 22) have a plane of symmetry including a direction of extension of the link (2).

5. The positioning support (1) according to any of the above claims,
wherein the link (2) has a radius of curvature comprised between 0.2 and
0.5 meters (for reaching same with the heel down and by a single swivel).

6. The positioning support (1) according to any of the preceding claims,
wherein the elongated link (2) has different indexings (215) at each of said fastening locations (200).

7. The positioning support (1) according to any of the preceding claims,
wherein said fastening locations (200) have studs (211, 212) protruding along a direction perpendicular to the direction of the extension of the link.

8. The positioning support (1) according to any of the preceding claims,
wherein said fastening locations (200) have an identical shape.

9. A positioning support (1) according to any of the preceding claims,
wherein said link (2) has a face with a non-slip surface finish.

10. The positioning support (1) according to any of the preceding claims,
wherein the second surface is inclined with respect to the first surface at an angle of at least 10°.

11. The positioning support (1) according to any of the preceding claims, wherein said elongated link (2) has a length of at least 400 mm.

12. A system, including:
- a positioning support (1) according to any of the preceding claims;
- a control pedal nested into a first interface (31) of one of said fastening devices,
